Europäisches Patentamt

European Patent Office

Office européen des brevets

(19)

(11) **EP 0 848 058 A1**

(12) **EUROPEAN PATENT APPLICATION**

(43) Date of publication:
**17.06.1998 Bulletin 1998/25**

(51) Int. Cl.$^6$: **C12N 13/00**, A01C 1/00,
A01G 7/04

(21) Application number: **96308975.0**

(22) Date of filing: **11.12.1996**

(84) Designated Contracting States:
**AT BE CH DE DK ES FI FR GB GR IE IT LI LU MC
NL PT SE**
Designated Extension States:
**AL LT LV RO SI**

(71) Applicant:
**Vergara Piccaluga, Alvaro, Dr.
Vina de Mar (CL)**

(72) Inventor:
**Vergara Piccaluga, Alvaro, Dr.
Vina de Mar (CL)**

(74) Representative:
**Spall, Christopher John
BARKER, BRETTELL & DUNCAN
138 Hagley Road
Edgbaston Birmingham B16 9PW (GB)**

(54) **Method of regulating cell behaviour**

(57)     A method of regulating the behaviour of living cells is disclosed in which the frequency of electromagnetic radiation emitted by the cell membranes and the magnitude of the frequencies is measured, whereby a stimulating signal determined by said emitted frequency can then be applied to the cells to regulate their behaviour by mimicking the cells own emitted radiation. The method can be used to regulate to rate of growth of seeds or to provide tumour cell lines from the cells. Other uses are envisaged and disclosed.

Fig 3

## Description

This invention relates to a method of regulating the behaviour of living cells, and in particular relates to plant, vegetable and animal cells.

This invention also relates to an improved industrial procedure for providing energy to seed cells in order to help seeds to grow better. More particularly, the invention uses procedures to measure seed cells electromagnetic communication after which their language can be electronically imitated, sending them either alternating electric current or electromagnetic field and verifying if they have been correctly stimulated electronically, before being planted.

## BACKGROUND OF THE INVENTION-

In order to explain better the real meaning of the invention, it is fundamental to present the following data, well known by everybody in biophysics, but never adequately interpreted before my work in history.

The external cell membrane in animals and vegetables is composed of two protein layers (conductive) separated by a lipidic one (isolating). It is transversally crossed by pores through which ion currents travel outside and inside the cell, regulated by the sodium-potassium pump.

In 1972, S.Jonathan Singer and Garth Nicolson proposed a fluid mosaic model to explain biological membranes fundamental organisation.(See Science 175 (1972: 723).

My interpretation of the aforementioned facts- Two conducting layers separated by an isolating one act as a condenser.

Pores, interconnected at the level of the extra and intracellular fluids act as spires in a coil. Ions face a resistance to travel.

So, I conclude, we have an R-L-C circuit in parallel, which can transmit electromagnetic waves, a changing and dynamic one.

This suffices to understand why a cell can transmit electromagnetic waves. External cell membrane surface area and metabolic energy will condition frequency and potency of emission, frequently around khz and milivolts.

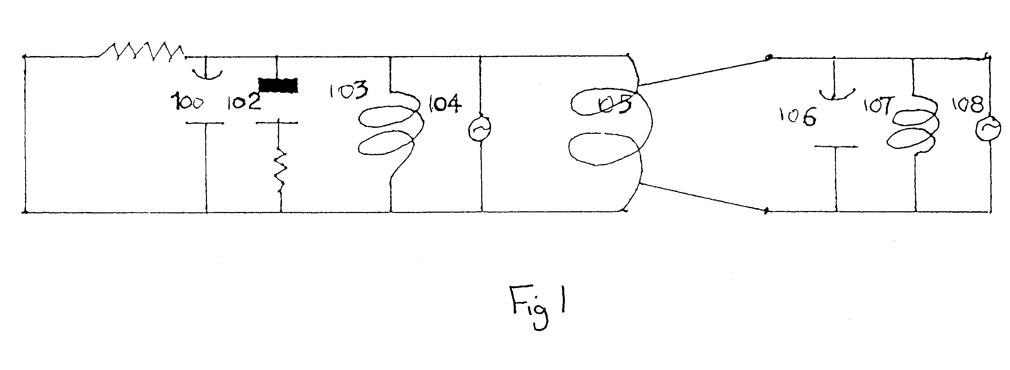

If we add to this that the external cell membrane R-L-C circuit rotates, cell nucleus also rotates and its membrane has a similar structure to the external cell membrane, that allows it to emit, we end up having a circuit of the type shown in Figure 1, where 101 is the condenser, 102 is the battery of the Sodium-potassium pump, 103 is the coil, 104 is the alternator, 105 is the autotransformer connection of the external cell membrane with the intracellular membrane system, 106 is the condenser in nucleus membrane, 107 is the coil nucleus membrane, 108 is the alternator function in nucleus.

## DESCRIPTION OF THE PRIOR ART-

Three patent applications could be cited as precedent ones if not dutifully examined:

a- UK Patent Application 2 159 390 A, published 4 Dec 1985, called A physical treatment for influencing biological activity by Stephen Von Mehesz. In his application he does not measure cell frequency in order to later imitate that behaviour. He does note that there are changes after passing some current through seeds immersed in water, but does not know why that happens. He says that the wave generated can be varied from 10 to 20 000 Hz, but he does not say according to what he changes frequency. In my registration the fundamental aspect of physiological stimulation is to provide the same frequency we first measure, to talk cells language. In line 20, page 3 of the specification he says' the modest electrical effects of the Mehesz Treatment can be envisaged as influencing the electron transfer processes of energy capture and release'. He seems to be guessing. In line 121 he says: ' The untreated ones scarcely hatched. The six treated eggs gave forth vigorous, healthy chicken. No explanation was possible'. His complete lack of notion of electromagnetic cellular communication is very clear.

In page 6 of the specification he says' It is also conceivable that the Mehesz effect works via electromagnetic boosting', etc. He again shows to be guessing. In line 60, page 6 he also guesses when saying 'The structure of the soil particles may be affected'.Nowhere in his claims he pretends to be detecting any cellular electromagnetic radiation and least of all to be imitating it or controlling its effects, the imitation.

b-SU 1553034 A 1 from Naximo Gorki Institute,Niv. N. I. Lobachevsky-Cuidad Gorki,Golubev-Giganov and Makin. In this application Voltage vibrations are amplified and they are caused by the movement of the liquid with ions, according to their interpretation their goal is to measure more precisely functional changes in plants, thanks to the detection of voltage vibrations changes, in order of microvolts. Using amplifiers they obtain milivolts and then they pass the signal on to spectrum analizer. The frequency measured goes from 0,1 to 30 Gigahertz, using other circuits. They measure higher frequency and lower voltage than I do. They never explain satisfactorily why vegetable cells emit this kind of radiation. Hardly could they use it to stimulate cells. c- FR-2618-291-A by Georgel J.P- 23-7-87, also published by Derwent Publications Ltd., 128, Theobalds Road, London WC1X8RP. He does not help cells in plants. 'The growing procedure monitors the variation of current emitted by the plants in response to their requirements for nutriment, temperature, light'. 'From this data the needs of the plants are determined and the growth conditions modified to adapt them to the measured needs of the plants. His treatment sends each plant electric currents in order to produce oxygen at the level of the roots and carbonic gas at the level of the leaves. He works with a differential signal and not with cells electromagnetic emission (see line 21 up to 25, page 4 in Procedure and device for vegetable reproduction). He measures resistivity changes and I measure

$$Fr = \frac{\cdot\ 1}{2\pi\sqrt{(C_1+C_2)(\frac{1}{L_1}+\frac{1}{L_2})}}$$

d- Froehlich electromagnetic field generated by living cells, computer results, by Pokorny J.; Vacek K.; Fiala J. from Department of chemical physics, Faculty of mathematics and physics, Charles University,121 16 Prague 2, Czechoslovakia. J Biol. Phys 12 (4) 1984 (RECD. 1985). 79-84.Coden JRPHBZ ISSN: 0092-0606. The Froehlich coherent polar vibrations may be a source of the cell-generated electromagnetic field. The cellular membrane is assumed to be the locus of the dipole vibrations. Computer results indicate that the electromagnetic field of cellular origin may be significant within a distance of about 1- 10 m$\mu$.m from the cell surface. The Schottky barrier might be used to determine the frequency and the intensity of the cell generated field in the frequency range < 1 Thz.

They are well above my frequency range and they say they measure molecular vibrations.

These authors have something in common: they ignore how cells really operate and why they emit electromagnetic waves so as they can receive them. I hold the patents to measure cellular electromagnetic radiation coming from the external cell membrane R-L-C circuit in parallel (numbers 123-23124- 23151, granted in Uruguay).

The new industrial procedure stimulates seeds with a vitally needed energy they cannot produce by themselves, not having their own foliage differentiated and solves the problem of productivity stimulation without contamination and the preservation of the seed. It gives seed cells physiologically the electromagnetic energy that need to grow before they can help themselves. It does so imitating cells language, that is their frequency of electromagnetic communication. After seed cells stimulation, it is possible to check if stimulation has been adequate, measuring resulting cellular emission and comparing it with previous data of stimulation V s. productivity.

This cellular electromagnetic transmission can be industrially applied:

A- catching it to use its energy;
B- influencing it directly and indirectly.

A- Electromagnetic energy canalization can allow electric energy generation by plants, communication by means of low intensity signals and as its consequence the construction of biological computers as well as bioelectronic prothesis.
B- Influences upon electromagnetic transmission can be carried out directly and specifically using electronic devices either to imitate cellular communication or to interfere with it ;
indirectly, modifying the transmitter;
unspecifically,e.g. by temperature changes.

These approaches, affecting intracellular transmission of the electromagnetic energy derived from the external cell membrane, can allow direct and indirect influence upon protein production, cells multiplication and dedifferentiation in for example, vegetable seed cells or animal cells.

In accordance with a first aspect of the invention, we provide a method of regulating the behaviour of one or more living cells having an external cell membrane characterised by the steps of:

measuring the frequency of electromagnetic radiation emitted by the cell membranes and the magnitude of said frequencies with electronic devices; and
applying a stimulating signal determined by said emitted frequency.

In accordance with a second aspect, we provide a method of regulating the rate of growth of seeds characterised by the steps of:

obtaining measurements indicative of the characteristic range of frequencies of electromagnetic radiation emitted by the seed cells within the seeds and the magnitude of said frequencies; and
applying a stimulating signal to the seeds, said stimulating signal being determined by said measured range of frequencies.

Several embodiments of the present invention will be explained, by way of example only, with reference to the accompanying drawings in which:

**Figure 1** shows an equivalent electrical circuit of the external cell membrane R-L-C network;

**Figure 2** shows the electrical circuit used to produce electric energy from a plant or vegetable or the like;

**Figure 3** is an illustration of a circuit for use in determining the range of frequencies of electromagnetic radiation emitted by a cell or cells;

**Figure 4** shows an alternative circuit suitable for inducing a current upon a conductor in order to determine the frequency and range of electromagnetic radiation emitted by the cell or cells;

**Figure 5** is an alternative circuit to the circuit shown in Figure 4 which works at lower frequencies; and

**Figure 6** is an enlargement of the transformer 12 shown in Figure 5.

**A- Example of electric energy production-**

Leaves in plants emit electromagnetic waves in a different frequency than branches and stem. This difference increases with solar illumination, thus generating a real dipole between either leaves and stem or leaves and ground. Connecting the points of different potential we can obtain an electromotive force.

**DESCRIPTION-**

Digital tester with amper meter and voltmeter, alternatively connected. The bases of leaves are connected in parallel to the positive pole of amper meter or voltmeter and the stem or ground to the negative pole.
Current so obtained (see following examples) can be amplified using the previous circuit:

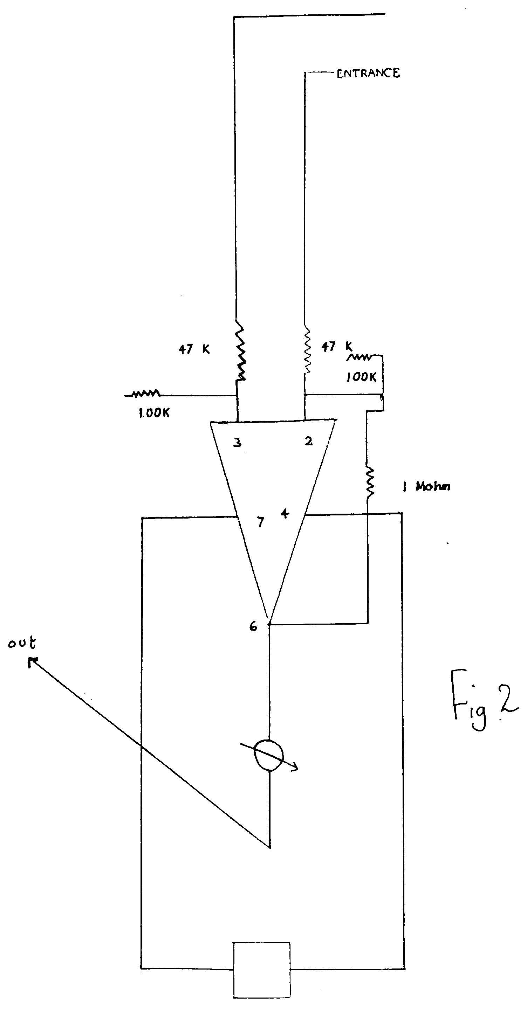

Legend for Figure 2-    At the entrance, signal from vegetable cells Output to oscilloscope, printer, etc.

The circuit includes operational amplifier and additional fountain.
When the operating frequency is known the adequate operational amplifier can be selected. It can also be employed to amplify continuous current.
Amplification with a transformer made to measure for the cellular group for a given frequency. It does not require an additional supply.
Knowing the operative frequency, two coils are made of enamelled wire, on the same axis, to increase magnetic coupling.
At high frequencies Z is practically equal to $X_1$,
R being = 0
$X_1 = 2\pi Fr.L$

$$L = \frac{X_1}{2\pi . Fr.}$$

In order to calculate the coil according to the operating frequency an empirical formula can be employed:

$$L \text{ in } \mu H = \frac{0.0787 . a^2 . n^2}{3a + 9b + 10c}$$

where a is height of the turns or diameter, n is number of turns, b is coil length and c is thickness of coil layers.

$$Fr^0 = \frac{1}{2\pi\sqrt{LC}}$$

Registration allows to obtain:

$L_1$, $C_1$ of the external cell membrane;
Fr. of the intracellular membrane system divisions;
$L_2$ and $C_2$ of those divisions and cellular electrons maximum kinetic energy .E.G.-

$$Fr^0 = \frac{1}{2\pi\sqrt{(C_1+C_2)(\frac{1}{L_1}+\frac{1}{L_2})}}$$

**EXAMPLE OF PRODUCTION OF ELECTRIC ENERGY BY VEGETABLES**

- Blinds low in the lab, at 2 p.m.

Variety measured with digital tester, for voltage and current intensity generated and with equipment to measure cell frequency.

Pothos (Scindapsus)   With and without ECG conducting paste.

**FREQUENCY**

| LEAF | STEM |
|---|---|
| a) 800 - 1000 Hz | a) 72 Hz |
| 45 - 105 Khz | 44 - 62 Khz |
| b) 900 - 1600 Hz | b) 1400 - 2000 Hz |
| 17500 - 46000 Hz | 50 84 Khz |
| c) 1450 - 1750 Hz | c) 154 Hz |
| 64 - 120 Khz | 16 - 38 Khz |
| d) 860 - 1020 Hz | d) 900 - 1000 Hz |
| 70 - 140 Khz | 52 - 90 Khz |
| e) 800 - 900 Hz | e) 740 - 1400 Hz |
| 76 - 160 Khz | 76 - 165 Khz |

**VOLTMETER (without paste)**

| AC | DC | Scale |
|---|---|---|
| 0 | -0,1 | 200 |
| 0,004 | -0,09 | 20 |
| 0,035 | -0,086 | 2 |
| 36 | -85,5 | 0,2 |

Ampermeter(in miliamper)- it does not produce any reading.
Raised blinds in the lab-same plant.
Time- 2 30 p.m. Direct sunlight over the plant.

**FREQUENCY**

| LEAF | STEM |
|---|---|
| a) 2000-2200 Hz | a) 9000-22000 Hz |
| 46-90 Khz | 6000-54000 Hz |
| b) 1250 Hz | b) 25-32 Khz |
| c) 4400-5200 Hz | c) 800-1250 Hz |
| 70-135 Khz | 36-74 Khz |
| d) 1750-2000 Hz | d) 260-300 Hz |
| 60-125 Khz | 60-90 Khz |
| e) 1000-1150 Hz | e) 84-150 Khz |
| 88-160 Khz | 1250-1500 Hz |

**VOLTMETER** (without conducting paste)

| AC | DC | Scale |
|---|---|---|
| 0 | -0,1 | 200 |
| 0,03 | -0,07 | 20 |
| 0,031 | -0,069 | 2 |
| 30,4 | -68,2-69,9 | 0,2 |

**AMPERMETER**

| AC | DC | Scale |
|---|---|---|
| 0 | 0 | 200 |
| 0 | 0 | 20 |
| 0,003 | 0 | 2 |
| 3,4 - 6,4 | 0,01 | 0,2 |

With paste in the contacts.

**VOLTMETER**

| AC | DC | Scale |
|---|---|---|
| 0 | 0 | 2000 |
| 0 | 0 | 200 |
| 0,07 | 0,01 | 20 |
| 0,007 | 0,011-0,012 | 2 |
| 7,8 | 10,5-13,5 | 0,2 |

**AMPERMETER**

| AC | DC | Scale |
|---|---|---|
| 0 | 0 | 2000 |
| 0 | 0 | 200 |
| 0 | 0 | 20 |
| 0,003 | 0 | 2 |
| 3,1 | 0,1 | 0,2 |

Knowing the frequency in AC, the current can be efficiently amplified.

**Method of regulating seed cell behaviour**

Applicant discovered that external electromagnetic energy can regulate seed cells behaviour, because they communicate by means of electromagnetic waves. As a consequence, he decided to provide that energy to the dormant cells that need it badly in seeds, using electronic devices. Acting in this way he respects cells integrity, helping them. He neither interferes with their vitality nor with their surroundings, as other technologies do.

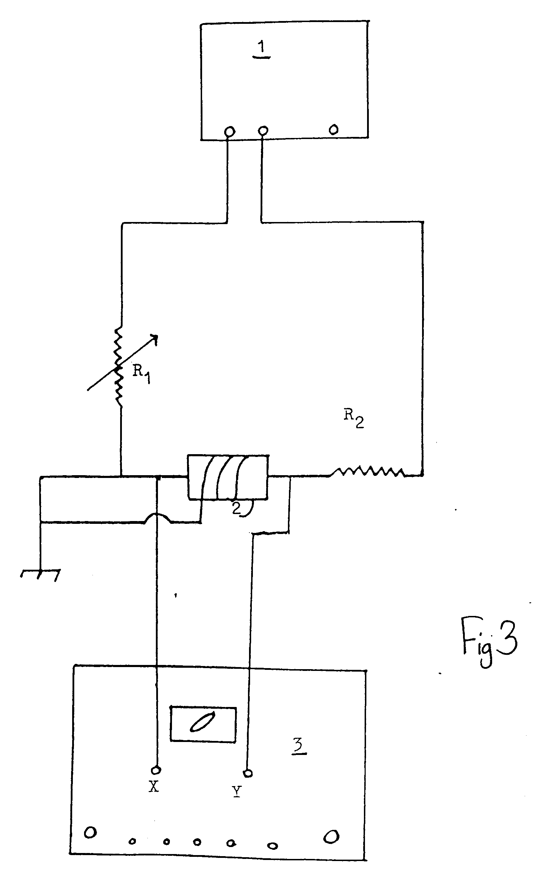

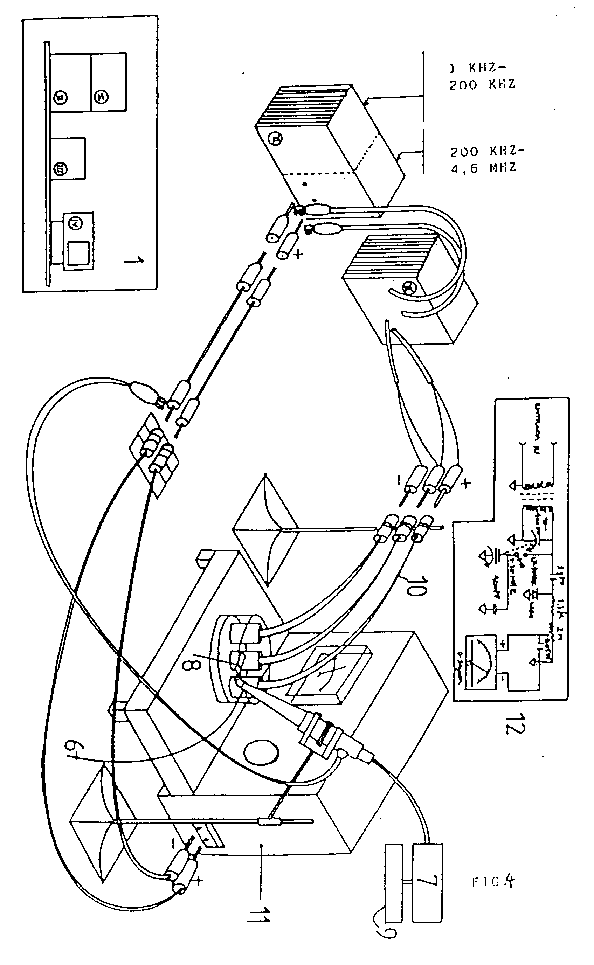

Figure 3 is a circuit where 1 is an electromagnetic waves generator(e.g. Lab Volt, Siemens); 2- is the group of cells traversed by electromagnetic waves; 3- is a double channel oscilloscope with x-y mode operating, e.g. kenwood, Trio 20 Mhz. Figure 4 is a circuit where I is a linear amplifier, acting from 200 Khz to 4,6 Mhz; II is a Lab Volt electromagnetic waves generator; III is an attenuator; IV is a double channel, 20 Mhz Kenwood oscilloscope; 6- Petri dish with a cell monolayer(which can be replaced by any cell group); 7- RF probe; 8-point of the RF probe (Fluke-100 Mhz);

9- digital tester;
10- conductors;
11- Electromagnetic field emitting regulated coil;
12- regulated coil plan.

Conductors end up in barrel like structures made from agar and additioned culture media, which stand on a monolayer.

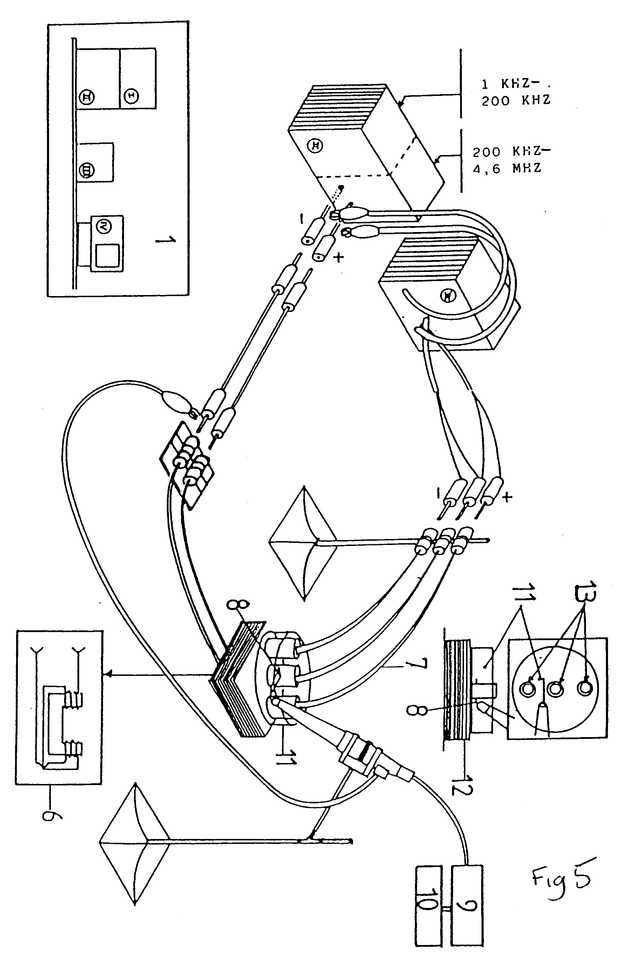

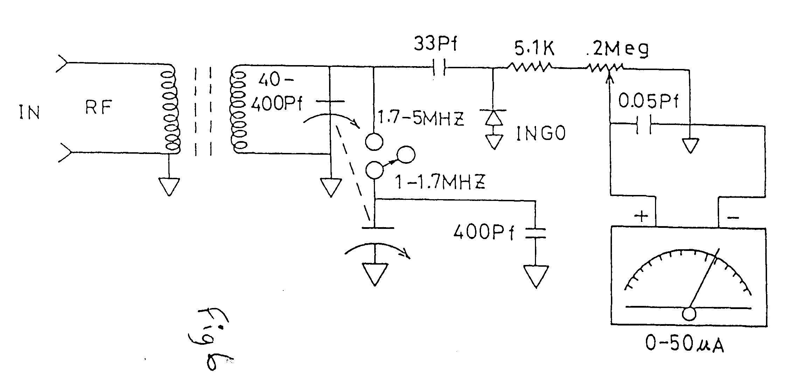

Figure 5,6-I,II,III, and IV like in Figure 5;

6-open nucleus transformer that emits electromagnetic field;
7-conductors, contacting cells in monolayer by means of the agar points, prepared with the cell culture media to render them conductive;
8-RF probe point;
9-RF probe;
10- digital tester;
11- cells monolayer;

12- transformer;
13- agar points cut transversally.

## Detailed description of the preferred embodiment-

The present invention provides a procedure to increase productivity of seeds physiologically, stimulating their cells either with electric current or electromagnetic field. Applicant was the first to discover that cells communicate by electromagnetic waves and because of that they can be affected in their growth by electromagnetic energy. In order to do so he proceeds by steps: step 1- cellular electromagnetic communication measurement, which is performed using;

I-Circuit in Figure 3, which operates by phase coincidence between an electromagnetic wave going from the generator to one channel of a double channel oscilloscope and the same electromagnetic wave, crossing cells and going to the other channel, when the X-Y mode is operating. When cells frequency coincides with the one of the electromagnetic wave we send from the generator, we have phase coincidence. Seed cells are usually contacted by metallic electrodes with conducting paste at their end;
II-Circuit in Figure 4, which shows the induction of the most intense electromotive force upon a conductor placed after the cells, when frequencies of electromagnetic field and traversed cells coincide. Field is generated by a coil and it can be precisely regulated working around 1Mhz or more in frequency;
III-Circuit in Figure 5, which works similarly to the previous one, but the field generated by the transformer operates at lower frequencies than the previous coil;
IV-Direct electromagnetic detection by conducting screens and an amplifying stage. This procedure gives an idea of emissary potency, in the sense that induced current intensity is inversely proportional to the distance that separates emitter and receptor and directly proportional to emitting potency.
Step 2-Once cells frequency is identified, seeds can than be electronically stimulated by apparatuses acting in their own frequency, graduating potency according to production desired, using either electromagnetic field or electric current. The use of this last one sometimes requires reduction of seed cover impedance, e.g. by hydrating it.
Step 3-Verification of seeds correct stimulation by measuring cellular electromagnetic communication (which is going to reflect the energetic change) resulting after stimulation, with procedures described in step 1.

Results are compared with lab standards of stimulation Vs. production, e.g. here I enclose an example of application, carried out at the Instituto Naeional de Investigaciones Agropecuarias, Las Brujas, Uruguay, with red garlic seed.
In that case I first measured cellular frequency in the seeds with circuit shown in Figure 3. It went, in those seeds, from 7 to 70 Khz; seeds were contacted by electrocardiogram conducting paste which separated them from diagnostic metallic electrodes, with three points, a central one to derive superficial waves to earth and two side, active ones to catch inner electric changes. Frequency measured in those seeds went from 7 to 70 Khz;

second-seeds were stimulated in their own frequency, with a Lab Volt generator, at 15 volts peak to peak, during 15 minutes, being contacted by copper electrodes. Voltage and time of exposure employed were previously determined in the lab with other similar seeds. Mean Z = 3M. Energy to be absorbed by each seed will depend upon impedance of its cover, different in each case. That's why the ideal way to act is individually, seed by seed and so did the author in this experiment, but acting with an industrial point of view it could be accepted to stimulate several seeds at a time;
third-I verified that each seed had been dutifully stimulated by measuring its resulting signal, e.g. using the circuit in Fig. 3 later comparing it with results obtained in previous seeds (in Uruguay example, mentioned here, I observed widening of the bandwidth);
fourth- seeds were planted without fertilizers, without pesticides and were watered.

Results-

Control - Weight-73 gr     Head diameter-57,53 mm
Treated - Weight-82 gr     Head diameter- 64,31 mm

In Chile, I also performed two additional demonstrations: in Chilean, Paine valleys, using red garlic seed provided by local producers. Both results also showed a 10 percent increase in yield.
Atlantic variety potatoes were also tested in Uruguay, with a 50% increase.
Water melon seeds were stimulated, obtaining a higher development speed.

B- ANIMAL EXAMPLES-

Example of protein production regulation-

Cells present an electromagnetic emission towards their outside and their interior. This part of the emission regulates all basic cellular functions, including antiparallel DNA chains separation and the subsequent protein synthesis that takes place.

If we modify external cell membrane emission frequency, we can regulate at will cellular protein production, since we shall be acting on DNA presentation.

MATERIALS EMPLOYED-

Two boxes with eight seven day old mice in each, with their mothers. One box receives 0,1 cc Intralipid subcutaneously during ten days. The other conditions are similar for both boxes. After this period the mice are killed by a blow and pancreas and liver in the two boxes are measured. This is followed by a homogenization with a mixer in distilled water. Previous exact precision weighting of the different groups of organs is carried out. If frequency does not change, as in liver cases, protein production does not change either.

C.P.-Control Pancreas
C.L.- Control Liver
L.P.- pancreas in mice with lipids
L.L.- Liver in mice with lipids

MICE WITH LIPIDS (Intralipid)

| LIVER | |
|---|---|
| 1- 150 to 4500 Hz | 5- 150 to 4200 Hz |
| 2- 150 to 4000 Hz | 6- 150 to 5000 Hz |
| 3- 150 to 9000 Hz | 7- 150 to 4400 Hz |
| 4- 150 to 8900 Hz | 8- 150 to 7000 Hz |
| MEAN Max. - 5875 Hz | MEAN Min. - 150 Hz |

| PANCREAS | |
|---|---|
| 1- 150 to 4500 Hz | 5- 150 to 3200 Hz |
| 2- 150 to 4400 Hz | 6- 150 to 8600 Hz |
| 3- 150 to 2600 Hz | 7- 150 to 5200 Hz |
| 4- 150 to 11000 Hz | 8- 150 to 6600 Hz |
| MEAN HIGH- 5775 Hz | LOW- 150 Hz, |

MICE WITHOUT LIPIDS (CONTROL)

| LIVER | |
|---|---|
| 1- 150 to 10500 Hz | 5- to 3500 Hz |

(continued)

| LIVER | |
|---|---|
| 2- 150 to 7000 Hz | 6- 150 to 4800 Hz |
| 3- 150 to 6900 Hz | 7- 150 to 8800 Hz |
| 4- 150 to 6200 Hz | 8- 150 to 5000 Hz |
| MEAN HIGH - 6587,5 Hz | LOW - 150 Hz |

| PANCREAS | |
|---|---|
| 1- 150 to 7400 Hz | 5- 150 to 8900 Hz |
| 2- 150 to 8400 Hz | 6- 150 to 3200 Hz |
| 3- 150 to 11500 Hz | 7- 150 to 12500 Hz |
| 4- 150 to 8000 Hz | 8- 150 to 8000 Hz |
| MEAN HIGH - 8487,5 Hz | LOW- 150 Hz |

WEIGHT CONTROL LIVER - 3,41 Grs.
WEIGHT LIVER OF MICE WITH SUBCUTANEOUS LIPIDS- 3,43 Grs.
WEIGHT CONTROL PANCREAS- 0,78 Grs
WEIGHT PANCREAS OF MICE WITH SUBCUTANEOUS LIPIDS- 0,63 Grs.

The different groups of organs are separately homogenized in distilled water with a mixer. They are then centrifuged in cold atmosphere at 5000 RPM during 20 min.
They are thawed at - 70 degrees and passed to Castro-Gherardi laboratory to measure proteins.

**EXAMPLE OF DEDIFFERENTIATION AND CELL MULTIPLICATION RHYTHM REGULATION**

Cellular dedifferentiation requires in order to take place a change in frequency and potency in the external cell membrane emission together with a change in the conduction of the voltage vibration from the external cell membrane to the intracellular general membrane system, what we are going to call electromagnetic coupling.
For frequency and potency to change, C and L in the external membrane

$$(Fr = \frac{1}{2\pi\sqrt{LC}} \text{ and } P = 1/2 \, L. \, I^2)$$

must change. Both are directly affected by the amount of lipids in the condenser dielectric and by proteins in its conducting layers.
When cells are subject to a functional hyperstimulation, e.g. cells in the adrenal cortex under the effect of an ACTH producing adenome, their external cell membrane works harder and their metabolism also does. This leads to an increase in the activity of the membrane condenser, which must bear a bigger movement of electric load, what is due to pores higher activity, with the subsequent increase of L, or even more pores activated.
The external cell membrane function elevation is due to an increase in the sodium-potassium pump activity, which will become more active, consuming more energy, obtained from cellular metabolism.
Let us remember here that antiparallel DNA chains are kept together by H bridges, where the electron, instead of covering its habitual orbit has to cover a broader one around O and H or H and N. This electron can be stimulated by the electromagnetic energy. The bridge can break and DNA chains can separate. When separated they can duplicate (theoretically a reduction in electromagnetic stimulation can also produce DNA separation, when it is below a threshold).
The increase in C and L lowers frequency and increases emissory potency in the external cell membrane, what increases antiparallel DNA chains disturbance, so facilitating their separation and increasing protein synthesis. This increase, provides protein molecules in bigger amount, which will store more load.

As the R-L-C circuit in the membrane works more than before, an isolation improvement is required, what prevents short-circuits between condenser plaques. This can be done in two successive steps:

1- increase in saturated fatty acids, without thickness increase, what reduces fluidity, slowing rotation speed, so reducing losses at pores level;

2- increasing dielectric thickness. The first raises C and lowers frequency, without changing L too much and the second reduces C and raises frequency and inductance (L). Electromagnetic coupling between external cell membrane and intracellular general membrane system, increases in percentage, as movement slows down.

Under this point of view of variations in degree in the electrical phenomenons which are the basis of cell metabolism, it is clear that the difference between a functional hyperstimulation (as in a hyperthyroidism or an adrenal adenoma) and a neoplasia is only a matter of degree. It is enough to prolong cell effort, varying C and L in the external cell membrane in their component parameters, during the necessary period of time, to produce neoplasia. This shows how to control appearance of neoplasia and how to prevent it from occurring.

MATERIAL AND METHODS-

3 boxes with adult rats, with six each, initially. All rats and mothers.

Box 1- Fed with the usual diet of the lab.

Box 2- Fed with the usual diet of the lab.

Box 3- Fed with the usual diet of the lab PLUS meat with fat and whole milk.

| 1 | 2 | 3 |
|---|---|---|
| 5-12-90 Nothing | 0,05 ml subcutaneous | ACTH 0,05 ml subcutaneous |
| 10-12-90 Nothing | 0,05 ml subcutaneous | ACTH 0,05 ml subcutaneous |
| 17-12-90 Nothing | 0,05 ml subcutaneous | ACTH 0,05 ml subcutaneous |
| 24-12-90 Nothing | 0,1 ml subcutaneous | ACTH 0,1 ml subcutaneous |
| 27-12-90 Nothing | 0,1 ml subcutaneous | ACTH 0,1 ml subcutaneous |
| 31-12-90 Nothing | 0,05 ml subcutaneous | ACTH 0,05 ml subcutaneous |

ENTIRE ADRENAL GLANDS MEASUREMENTS- Box 1 (3-1-91)

| RIGHT | LEFT |
|---|---|
| 1-120 to 200 Khz | 20 to 200 Khz |
| 2-64 to 200 Khz | 3400 Hz to 200 Khz |
| 3- 9 to 200 Khz | 50 to 200 Khz |
| 4- 10 to 200 Khz | 30 to 200 Khz |
| Mean - 38300 Hz to 200 Khz | |
| Mean - Range 161.700 Hz | |
| Box 2 | |
| 1- 1650 Hz to 11500 Hz | 1500 Hz to 30.000 Hz |
| 2- 3100 Hz to 20.000 Hz | 5.000 to 12500 Hz and 66 to 200 Khz |
| 3- 4600 Hz to 200 Khz | 1100 to 200.000 Hz |
| 4- 8100 to 200.00 Hz | 64 to 200 Khz |
| 5- 46 to 200 Khz | 1000 to 28.000 Hz |
| 6- 3400 Hz to 200.000 Hz | 10 to 200 Khz |
| Mean- 12454,166 Hz to 140791,66 Hz | |
| Mean Range- 128337,5 Hz | |
| Box 3 | |
| 1- 5 to 200 Khz | 135 to 200 Khz |
| 2- 96 to 310 Khz | 54 to 200 Khz |
| 3- 2 to 200 Khz | 3300 to 200.000 Hz |
| 4- 2900 to 200.000 Hz | 110 to 200 Khz |
| 5- 1550 to 200.000 Hz | 750 to 11.000 Hz |
| Mean- 41050 to 192100 Hz | |
| Mean Range- 151050 Hz | |

PATHOLOGICAL REPORT MATERIAL; RAT ADRENAL CORTEX. INITIAL FIXATION WITH BOUIN SOLUTION DURING SIX HOURS AND THEN FORMALDEHYDE 10%.

Control box laminae-Adrenal gland sections composed of cortex with the three representative layers and medullar layer. Cortical cells show egg-shaped, monomorphic nuclei and an eosinophillic citoplasm, intensely granular and compact.

Laminae from the box with subcutaneous ACTH (2) -Adrenal gland sections composed of cortex with a thickness similar to that in box 1, with the three representative layers and a monomorphic nucleus. The fasciculated layer presents cells of a clearer aspect, as though depleted. A little more congestion than normal is observed.

Laminae from box with ACTH plus special diet-(3)- Thicker cortex is seen, than in the other boxes. Cells with two nuclei can be seen. Compact citoplasm slightly granular, but much less so than in box containing ACTH alone, in some laminae. Reticular layer can be more clearly seen. More cells can be seen in general.

INTERPRETATION-

ACTH lowers frequency, raising C and L. Dietary fat (proteins in excess also go to create fat) raises dielectric thickness, lowering C and raising L.

ACTH and diet compensate their mutual effects on adrenal gland emission frequency. In order to keep box 3 frequency similar to box 1, with lipids in the membrane elevated (which in the end lowers C) an increase in C by a larger area is required

$$C = E \frac{A}{d} \cdot )$$

So, there being a similar frequency with a larger external cell membrane area, there will be a broader surface irradiating towards cell interior and DNA will be more excited, together with the rest of the cell, because of this, cells are in general more emptied of their production.

With larger emission surface in the external cell membrane, thicker dielectric and more saturated acids in the latter, rotation speed decreases in the fluid layer and distance increases.

Also when dielectric thickness increases, transversal pressure on adjacent protein layers increases, so friction increases.

As Energy increases (potency) and electromagnetic stator-rotor percentage coupling grows, a higher external cell membrane energy is more efficiently transmitted to the intracellular membrane system.

## Claims

1. A method of regulating the behaviour of one or more living cells having an external cell membrane characterised by the steps of:

   measuring the frequency range of electromagnetic radiation emitted by the cell membranes and the magnitude of said frequencies with electronic devices; and
   applying a stimulating signal determined by said emitted frequency.

2. A method according to claim 1 in which said stimulating signal comprises an alternating electric current applied at a variable voltage and a frequency variable up to 2Mhz.

3. A method according to claim 1 in which said stimulating signal comprises electromagnetic radiation of variable potency and a frequency variable up to 2Mhz.

4. A method according to any preceding claim in which said stimulating signal is substantially the same as a said measured frequencies of electromagnetic radiation emitted by said cell or cells.

5. A method according to any one of the preceding claims comprising the additional step of measuring the resulting characteristic range of frequencies and magnitude of the electromagnetic radiation emitted by the cells after said application of said stimulating signal.

6. A method according to any preceding claim comprising the additional step of identifying a correlation between the applied stimulating signal and the resulting change in behaviour of the cells.

7. A method according to any preceding claim in which said cells comprise either vegetable, e.g. seed cells or animal cells.

8. A method of regulating the rate of growth of seeds in number, size, weight and development speed of the resulting plant characterised by the steps of:

   obtaining measurements indicative of the characteristic range of frequencies of electromagnetic radiation emitted by cells within the seeds and the magnitude of said frequencies; and
   applying a stimulating signal to the seeds, said stimulating signal being determined by said measured range of frequencies.

9. A method according to claim 8 in which said stimulating signal comprises an alternating electric current and in which said seed covers of said cells are at least partially dehydrated.

10. A method according to claim 8 comprising the further step of measuring the resulting characteristic range of frequencies after the application of said stimulating signal to said seeds, before planting said seeds.

**11.** A method of producing tumor cell lines characterised by directly applying a stimulating signal to a plurality of cells in which said stimulating signal imitates the frequency of cellular electromagnetic emission of said cells.

**12.** A method of generating electric energy from a plant comprising branches, stems and leaves supported on said branches and stems characterised by the steps of connecting at least two parts of said plant having different potential into an electric circuit to produce an electric signal, and amplifying said electric signal.

**13.** Industrial procedures that catch and influence cellular electromagnetic transmission, characterised in that the induction of an electric functional change in frequency, potency and electromagnetic coupling between external cell membrane and intracellular general membrane system, are accompanied by changes in protein synthesis, cell multiplication and dedifferentiation.

**14.** Industrial procedures that catch and influence cellular electromagnetic transmission in an indirect way, characterised in that frequency, potency and electromagnetic coupling found are modified at will, varying cellular membrane components by means of diets, drugs, physical measures.

**15.** Industrial procedures that catch and influence cellular electromagnetic transmission, characterised in that frequency detection of the R-L-C external cell membrane circuit, connected via autotransformer connection to the intracellular L-C, of the external cell membrane ,L,C, provides individual frequencies of intracellular membrane dependencies and intracellular electrons maximum kinetic energy.

**16.** Industrial procedures that catch and influence cellular electromagnetic transmission, characterised in that they work detecting frequencies lower than 2Mhz and voltages in the order of microvolts and millivolts.

**17.** Industrial procedures that catch and influence cellular electromagnetic transmission characterised because they can be allowed to alter cellular function and structure, either in a gradual or craggy way, till if is destroyed, what can be industrially used eliminating contaminant cells in cell cultures.

**18.** Industrial procedures that catch and influence cellular electromagnetic transmission, characterised because the change of frequency potency and electromagnetic coupling induced by an agent can either be prevented by the action of another one that acts in the opposite way or reverted.

**19.** Industrial procedures that catch and influence cellular electromagnetic transmission in accordance with any proceeding claim, characterised because cells electromagnetic frequency is measured by phase coincidence between an electromagnetic wave sent from a generator to one channel, in a double channel oscilloscope and the same wave going directly to the other channel, with the X-Y mode operating, contacting cells by means of three points electrodes with conducting paste at their ends.

**20.** Industrial procedure that catch and influence cellular electromagnetic transmission in accordance with any proceeding claim, characterised because cells frequency is measured by induction of the most intense electromotive force on a conductor placed after the cells, when frequency of an electromagnetic field generated by a coil coincides with cellular electromagnetic frequency, placing cells between field emitter and induction receiver.

**21.** Industrial procedure that catch and influence cellular electromagnetic transmission in accordance with any proceeding claim, characterised because cells electromagnetic frequency and potency are measured by direct detection with either conducting screens placed near them or with contacting metallic electrodes, where the current induced on the screen is inversely proportional to its distance from cells and directly proportional to emission potency, being after direct detection dutifully amplified by linear amplifiers.

Fig 1

Fig 2

Fig 3

1 KHZ-
200 KHZ

200 KHZ-
4,6 MHZ

FIG 4

18

1 KHZ-
200 KHZ

200 KHZ-
4,6 MHZ

Fig 5

Fig 6

European Patent
Office

# PARTIAL EUROPEAN SEARCH REPORT

which under Rule 45 of the European Patent Convention
shall be considered, for the purposes of subsequent
proceedings, as the European search report

Application Number

EP 96 30 8975

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DE 32 44 582 A (MERSMANN LUDGER) 20 December 1984<br>* the whole document *<br>* page 26, paragraph 3 *<br>* page 27, paragraph 3 *<br>* page 28 *<br>--- | 1-21 | C12N13/00<br>A01C1/00<br>A01G7/04 |
| X<br>Y | EP 0 533 585 A (ZHOU LIN) 24 March 1993<br>* page 5, line 55 - page 6, line 34 *<br>* examples 23-25 *<br>--- | 1-21<br>1-21 | |
| X,D | FR 2 618 291 A (GEORGEL JEAN PIERRE) 27 January 1989<br>* page 4, line 32 - line 34; claims *<br>--- | 1,2,4-7,<br>11-14,21 | |
| X | DATABASE WPI<br>Derwent Publications Ltd., London, GB;<br> AN 97-019180<br>XP002032390<br>* abstract *<br>& RU 2 057 420 A (GOLD EG STOCK CO) 10 April 1996<br>---<br>-/-- | 1,3,4,<br>6-8,<br>13-21 | |

| TECHNICAL FIELDS SEARCHED (Int.Cl.6) |
|---|
| C12N<br>A01C<br>A01G<br>C12C |

## INCOMPLETE SEARCH

The Search Division considers that the present European patent application does not comply with
the provisions of the European Patent Convention to such an extent that it is not possible to carry
out a meaningful search into the state of the art on the basis of some of the claims.

Claims searched completely :

8-21

Claims searched incompletely :

1-7

Claims not searched :

Reason for the limitation of the search:

Remark: Athough claims 1-7 (as far as in vivo methods
are concerned) are directed to a method of treatment
of the animal body, the search has been carried out
and based on the alleged effects of the treatment

| Place of search | Date of completion of the search | Examiner |
|---|---|---|
| THE HAGUE | 9 June 1997 | Coucke, A |

EPO FORM 1503 03.82 (P04C07)

**European Patent Office**

## PARTIAL EUROPEAN SEARCH REPORT

Application Number

EP 96 30 8975

| | DOCUMENTS CONSIDERED TO BE RELEVANT | | CLASSIFICATION OF THE APPLICATION (Int.Cl.6) |
|---|---|---|---|
| Category | Citation of document with indication, where appropriate, of relevant passages | Relevant to claim | |
| Y | WO 87 02705 A (SWEENEY GEORGE WILLIAM JR) 7 May 1987 * page 1, line 23 - page 3, line 5; claims; example 4 * * page 16, line 20 - page 17, line 23 * --- | 1-21 | |
| X | EP 0 039 163 A (ELECTRO BIOLOGY INC) 4 November 1981 | 1 | |
| Y | * the whole document * --- | 1-21 | |
| X | GB 417 501 A (JAMES YATE JOHNSON) 28 September 1934 * page 8, line 119 - page 9, line 40; claims * * page 10, line 44 - line 92 * --- | 1,3,4, 6-8,11, 13-21 | |
| X | WO 96 36207 A (MOROZOV NIKOLAI FEDOROVICH ;MOROZOV NIKOLAI NIKOLAEVICH (RU); CHET) 21 November 1996 * abstract * & DATABASE WPI Week 97 Derwent Publications Ltd., London, GB; Class 01, AN 97-011733 * abstract * --- | 1,3,4, 6-8, 13-16 | |
| E | DATABASE WPI Section PQ, Week 9721 Derwent Publications Ltd., London, GB; Class P11, AN 97-229944 XP002032388 & JP 09 070 205 A (VERGARA PICCALUGA A) , 18 March 1997 * abstract * ----- | 1-21 | |

**TECHNICAL FIELDS SEARCHED**    (Int.Cl.6)

EPO FORM 1503 03.82 (P04C10)